# EUROPEAN PATENT APPLICATION

(11) **EP 3 560 420 A1**
(43) Date of publication of application: **30.10.2019**
(21) Application number: 19170929.4
(22) Date of filing: 24.04.2019
(51) Int. Cl.: A61B 5/0215, A61B 5/021, A61B 5/022, A61B 5/024, A61B 5/053, A61B 17/11, A61B 17/12

(54) **SURGICAL DEVICE INCLUDING SYSTEMS FOR SENSING TISSUE PROPERTIES AND METHODS THEREOF**

(30) Priority: 25.04.2018 US 201862662242 P; 30.01.2019 US 201916261962
(71) Applicant: Covidien LP, Mansfield, MA 02048 (US)
(72) Inventor: WILLIAMS, Justin, Southbury, CT 06488 (US)
(74) Representative: Soames, Candida Jane

(57) **Abstract**

Systems and method for sensing a tissue property with a surgical device are disclosed. The surgical device may include a handle, an outer shaft coupled to the handle, an inner shaft slidably coupled to the outer shaft, and a sensing assembly coupled to a distal portion of the outer shaft. The sensing assembly may include a shuttle having an anvil and a sensor disposed on the anvil, a compression housing coupled to the distal portion of the outer shaft, an expansion spring disposed between the shuttle and the compression housing, and a release coupled to the shuttle and biased to engage a fluid conduit. The compression housing may be located proximal to the anvil. The release may be configured to fix the position of the shuttle relative to the outer shaft.

## Description

### CROSS-REFERENCE TO RELATED APPLICATION

This application claims the benefit of and priority to U.S. Provisional Patent Application No. 62/662,242 filed April 25, 2018, the entire disclosure of which is incorporated by reference herein.

### BACKGROUND

### Technical Field

The present disclosure relates generally to surgical devices and, more particularly, to systems and methods for measuring one or more tissue properties during a surgical procedure.

### Related Art

During surgical procedures medical professionals or clinicians may find it desirable to determine one or more tissue properties prior to acting upon the tissue. For example, during colorectal surgeries which require anastomosis, the clinician visually inspects the tissue of the colon to be resected. Typically, during inspection, the clinician visually observes the colon and determines which portion or portions of the colon are diseased. The clinician then identifies which diseased portions of the colon will be removed. Observation may be performed via one or more imaging devices positioned within the colon or proximate to the colon. Various other surgical procedures require similar visual inspection of tissue to determine which portions of tissue are to be removed.

Depending on the procedure and the tissue being examined, the clinician may not identify all areas of concern due to the limited visibility of the clinician. For example, referring again to anastomotic procedures, the colon may include an abnormal growth which may not be easily visualized from an inspection of the exterior of the colon. As such, the clinician may need to inspect the interior of the tissue to be resected as well. Inspection of the interior of the colon may require additional clinicians to assist in imaging the interior of the colon. Additionally, care must be taken when aligning the interior and exterior views during the imaging process.

As such, improved systems and methods for evaluating tissue properties during a surgical procedure are desirable.

### SUMMARY

Existing challenges associated with the foregoing, as well as other challenges, are overcome by methods for identifying one or more properties of target tissue, and also by systems, and apparatuses that operate in accordance with the methods.

According to embodiments of the present disclosure, a surgical device for sensing a tissue property is disclosed. The surgical device includes a handle, an outer shaft coupled to the handle, an inner shaft slidably coupled to the outer shaft, and a sensing assembly coupled to a distal portion of the outer shaft. The sensing assembly includes a shuttle having an anvil and a sensor disposed on the anvil, an expansion spring disposed between the shuttle and the compression housing, and a release coupled to the shuttle and biased to engage a fluid conduit, the release configured to fix the position of the shuttle relative to the outer shaft.

In aspects, the surgical device further includes a bladder coupled to the fluid conduit. The bladder may be configured to apply pressure to target tissue when fluid is introduced to the bladder.

According to aspects, the shuttle may be configured to transition from an open configuration to a closed configuration in response to longitudinal movement of the inner shaft.

In aspects, the release is configured to apply pressure to the fluid conduit to lock the shuttle in position relative to the outer shaft.

According to aspects, the pressure applied by the release to the fluid conduit is greater than the pressure exerted by the expansion spring to the shuttle.

In aspects, engagement of the release by the inner shaft when the shuttle is advanced distally causes the shuttle to advance proximally.

According to aspects, engagement of the release by the inner shaft when the shuttle is advanced distally causes the shuttle to advance proximally.

In embodiments of the present disclosure, a method of sensing a tissue property with a tissue property sensing device is disclosed. The method includes positioning a sensing assembly of a tissue property sensing device about target tissue, engaging a release arm to transition the sensing assembly from an open configuration to a closed configuration, compressing the target tissue disposed in the sensing assembly, and sensing a tissue property.

In aspects engaging the release arm causes an expansion spring to apply force to a shuttle to move the shuttle from a distal position relative to the tissue property sensing device to a proximal position.

According to aspects, the method may further include disengaging the release arm after the sensing assembly is transitioned to the closed configuration.

In aspects, disengaging the release arm causes the sensing assembly to lock in the closed configuration.

According to aspects, disengaging the release arm causes the sensing assembly to lock in the closed configuration.

In aspects, the method further includes engaging the release arm after sensing the tissue property, and transitioning the sensing assembly from the closed configuration to the open configuration. Engaging the release arm may cause the sensing assembly to unlock.

According to aspects, the method may further include decompressing the target tissue prior to transitioning the sensing assembly from the closed configuration to the open configuration.

In aspects, compressing may include increasing pressure in a balloon fixed to the sensing assembly.

According to aspects, the method may further include locking the sensing assembly when in the closed configuration. Compressing may include delivering fluid to the balloon to increase the pressure of the balloon when the sensing assembly is locked in the closed configuration.

### BRIEF DESCRIPTION OF THE DRAWINGS

The accompanying drawings, which are incorporated in and constitute a part of the specification, illustrate embodiments of the present tissue sensing devices and methods and, together with a general description of the disclosure given above, and the detailed description of the embodiment(s) given below, serve to explain the principles of the disclosure.
FIG. 1 is a perspective view of a tissue property sensing device in accordance with an embodiment of the present disclosure;
FIG. 2 is a side plan cross-sectional view of the tissue property sensing device of FIG. 1 taken along section line 2-2 of FIG. 1;
FIG. 3A is a detailed, side plan, view of a sensing assembly of the tissue property sensing device of FIG. 1 in an open configuration;
FIG. 3B is a detailed, side plan, view of the sensing assembly of the tissue property sensing device of FIG. 1 in an approximated configuration;
FIG. 4 is a detailed, side plan, view of an alternate embodiment of the tissue property sensing device and the sensing assembly of FIG. 1;
FIG. 5 is a schematic diagram of a computing device that may be employed according to various embodiments of the present disclosure; and
FIG. 6 illustrates a flow diagram showing an illustrative method for measuring a tissue property of target tissue.

### DETAILED DESCRIPTION

Embodiments of the present tissue sensing instruments or devices and methods are described in detail with reference to the drawings, in which like reference numerals designate identical or corresponding elements in each of the several views.

Reference will be made to the terms described herein while describing the principles outlined by the present disclosure. As used herein, the term "clinician" refers to a doctor, nurse, or other care provider and may include support personnel. The term "distal" refers to structure that is, in use, positioned farther from the clinician, while the term "proximal" refers to structure that is closer to the clinician. Further, directional terms such as front, rear, upper, lower, top, bottom, distal, proximal, and the like are used to assist in understanding the description and are not intended to limit the present disclosure. The term "surgical field" refers to the space in which the surgical procedure is performed, and the term "surgical cavity" refers to a cavity at least partially surrounded by tissue.

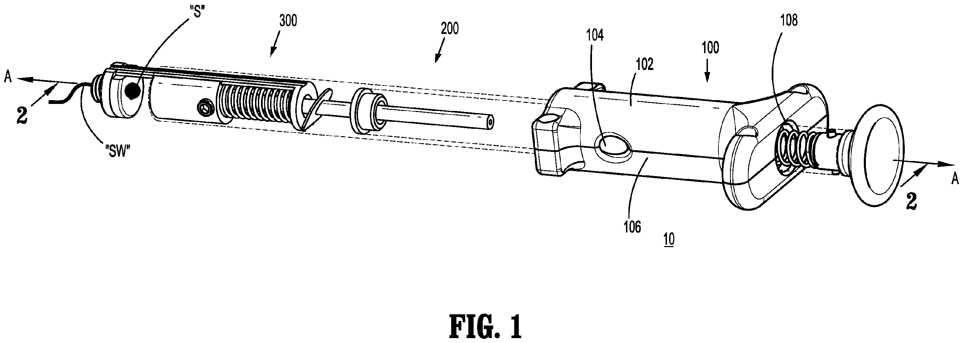

Referring initially to FIG. 1, one embodiment of a tissue property sensing device is shown and generally referred to as a surgical device 10. The surgical device 10 defines a longitudinal axis A-A and includes a handle 100, an actuation assembly 200 extending distally from the handle 100, and a sensing assembly 300 coupled to a distal portion of the actuation assembly 200. The handle 100 has a housing 102 configured to be gripped by the hand of a clinician. The housing 102 has an access aperture 104 extending through a side portion of the housing 102, the access aperture 104 configured to enable a fluid hose (not shown) to extend through the housing 102 to provide fluid (e.g., air) to a sensing assembly 300, discussed below. The housing 102 further includes a pair of apertures 108 which define a diameter of a bore extending from the proximal portion of the housing 102 to the distal portion along the axis A-A. The bore of the housing 102 extending along the axis A-A defines a lumen which is configured to be fixed about a proximal portion of an actuation assembly 200.

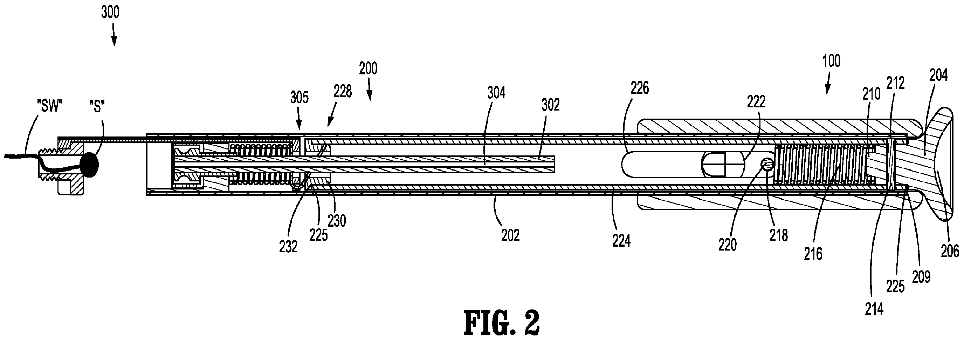

The housing 102 has a proximal portion and a distal portion configured to receive proximal and distal forces exerted by a clinician, respectively. The housing 102 is formed of a first and second clamshell, though in embodiments the housing 102 may be monolithically formed. The first and second clamshells may be coupled by any conventional means such as, for example, with one or more pins, screws, adhesives, welds, and the like. The lumen extending through the housing 102 is configured to receive a fluid hose (not shown) when inserted through the access aperture 104 extending through the side of the housing 102. The housing 102 further includes a pair of opposing recesses 106 configured to receive a fixation pin 220 (FIG. 2) therebetween.

Referring now to FIG. 2, the handle 100 (FIG. 1) is configured couple to a proximal portion of the actuation assembly 200. The actuation assembly 200 includes an outer shaft 202 having a pair of apertures 209 disposed along the proximal and distal portions thereof. The proximal and distal apertures 209 define a bore which extends through the outer shaft 202 forming a lumen extending along the axis A-A of the outer shaft 202. Additionally, the outer shaft 202 has an access aperture 222 disposed along a proximal side portion of the outer shaft 202, the access aperture 222 configured to align with the access aperture 104 of the handle 100. The outer shaft 202 further includes an opening 218 which is configured to receive a fixation pin 220 at least partially therein.

The outer shaft 202 is configured to slidably receive an inner shaft 224 having a pair of apertures 225 disposed along the proximal and distal portions of the inner shaft 224. The inner shaft 224 has a window 226 formed therein that extends along the inner shaft 224 beyond the extents of the access aperture 222 of the outer shaft 202. The window 226 of the inner shaft 224 is further configured to provide a mechanical limit for motion of the inner shaft 224 relative to the outer shaft 202. More particularly, as the inner shaft 224 is advanced distally, a proximal portion of the window 226 of the inner shaft 224 comes in contact with the fixation pin 220, thereby preventing continued distal motion of the inner shaft 224 relative to the outer shaft 202. Additionally, the window 226 of the inner shaft 224 is further configured to provide a mechanical limit for proximal motion of the inner shaft 224 relative to the outer shaft 202. Specifically, as the inner shaft 224 is advanced proximally a distal portion of the window 226 contacts the fixation pin 220, thereby preventing continued proximal motion of the inner shaft 224 relative to the outer shaft 202.

The proximal portion of the inner shaft 224 is configured to receive and couple to a knob 204. The knob 204 has an indent 206 configured to be engaged by the thumb of a clinician when the clinician advances the actuation assembly 200 distally. The knob 204 has a neck 208 extending distally therefrom. The neck 208 has a transverse bore configured to receive a fixation pin 214 therein. When the neck 208 of the knob 204 is inserted into the proximal portion of the inner shaft 224, the fixation pin 214 is inserted into at least one opening 212 disposed along, and in cooperative alignment with, the proximal portion of the inner shaft 224. The neck 208 has an alignment cylinder or nub 210 extending therefrom. The neck 208 is configured to engage a spring 216, with the alignment cylinder 210 maintaining the position of the spring 216 within the inner shaft 224.

The spring 216 extends distally from the neck 208 and engages the fixation pin 220 of the inner shaft 224. The fixation pin 220 extends through openings 218 formed along the inner shaft 224, and in opposed relation to one another. By default, the spring 216 applies longitudinally directed outward forces to the fixation pin 220 and the knob 204, thereby causing the knob 204, and more generally the inner shaft 224, to be biased proximally relative to the outer shaft 202.

The inner shaft 224 has an engagement ring 228 inserted into the distal portion thereof. The engagement ring 228 includes a collar 230 which extends proximally from the distal aperture 225 of the inner shaft 224 and an engagement surface 232 coupled distally to the collar 230. The engagement surface 232 has an opening formed therein and located centrally therealong. The opening is configured to receive a fluid conduit 302 therethrough. The engagement ring 228 may be coupled to the distal portion of the inner shaft 224 by any conventional means including, without limitation, being fixed with a pin, an adhesive, and the like. In embodiments, the engagement ring 228 is monolithically formed with the inner shaft 224.

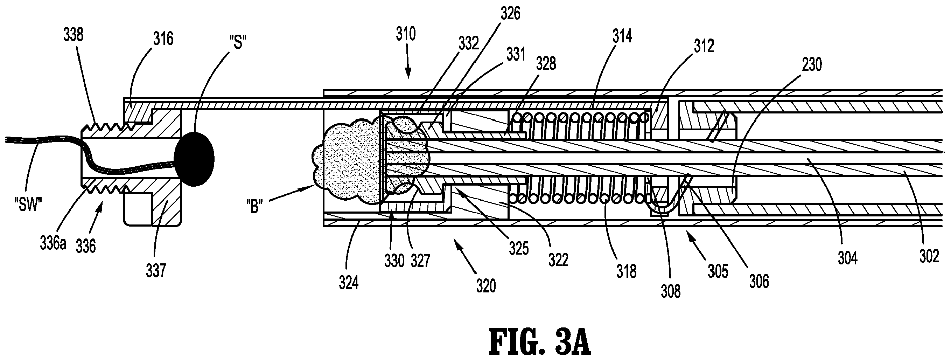

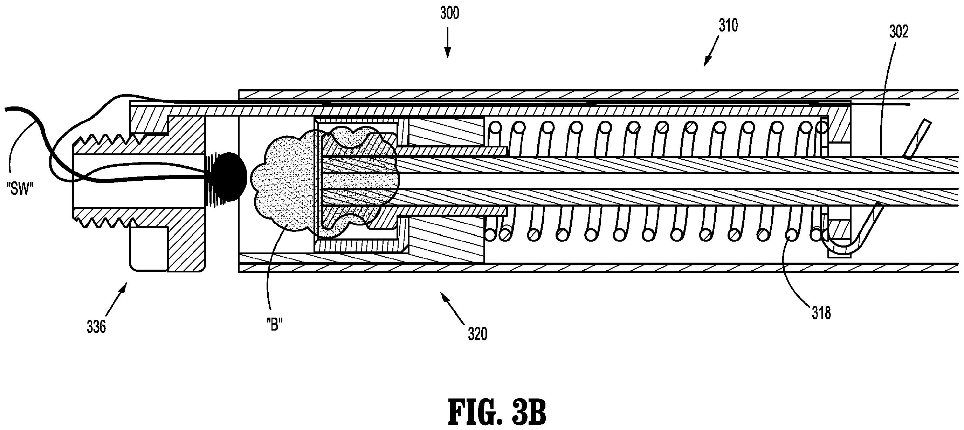

Referring now to FIGS. 3A and 3B, illustrated is a sensing assembly 300 of surgical device 10 configured to compress or otherwise engage target tissue. The sensing assembly 300 includes a shuttle 310 slidably received in the distal portion of the actuation assembly 200. The shuttle 310 is configured to move proximally and/or distally to compress target tissue. The shuttle 310 has a base 312, an arm 314 extending distally from the base 312, and a head 316 coupled to a distal portion of the arm 314. The arm 314 may be formed to receive the wired connector "SW" to couple a sensor "S" to a controller (not explicitly shown).

The base 312 and the head 316 of shuttle 310 have corresponding openings formed therein and located centrally along respective surfaces thereof. The opening of the base 312 is configured to slidably receive the fluid conduit 302 therein. The base 312 further has a release 305 coupled to a distal surface thereof. The release 305 includes a fixation arm 308 and an engagement arm 306 which are joined to monolithically form the release 305. The fixation arm 308 and the engagement arm 306 have openings formed therein and configured to slidably receive the fluid conduit 302 therethrough. The engagement arm 306 is biased proximally in an un-engaged configuration (FIG. 3B) which causes the engagement arm 306 to disable motion of the shuttle 310 relative to the fluid conduit 302. The opening of the engagement arm 306 forms an elliptical opening which, as the engagement arm 306 is advanced distally relative to the fixation arm 308, enables motion of the fluid conduit 302 through the release 305, and by extension, enables motion of the shuttle 310 distally relative to the outer shaft 202.

An expansion spring 318 is disposed between the release 305 and a compression housing 320 which is fixed to the inner surface of the outer shaft 202. The expansion spring 318 is configured to apply outward force (in a longitudinal direction) toward the release 305 (and by extension the base 312 of the shuttle 310) and a base 322 of the compression housing 320. Since the base 322 of the compression housing 320 is fixed relative to the outer shaft 202, the outward force exerted by the expansion spring 318 is received by the shuttle 310, thereby causing the shuttle 310 to be biased proximally when proximal and distal motion of the shuttle 310 is unimpeded by the release 305.

As mentioned above, the compression housing 320 includes a base 322, and a compression chamber wall 324. The compression chamber wall 324 extends distally from the base 322 of the compression housing 320 and encloses a cavity configured to receive a bladder "B" therein. A pressure sensor (not shown) may be disposed about the surgical device 10 to measure the pressure inside the bladder "B". The base 322 has an opening configured to receive a shaft 328 of a compression piston 325. The compression piston 325 is coupled to a distal portion of the fluid conduit 302. A piston housing 330 is fixably joined to the base 322 of the compression housing 320 and is coupled to a proximal portion of the compression chamber wall 324. The piston housing 330 may be coupled to the compression housing 320 via a friction fit, adhesives, or by any other suitable method of attachment.

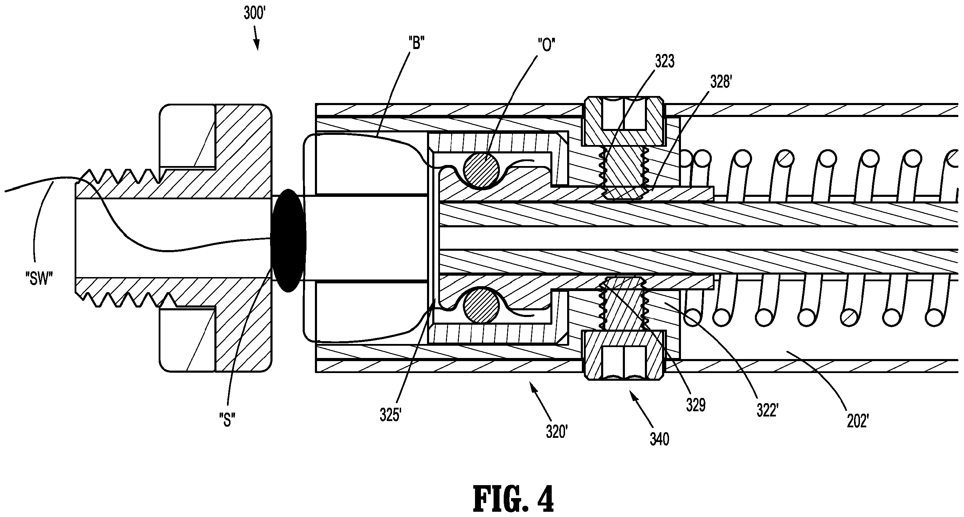

The compression piston 325 has a piston head 326 and a shaft 328 extending proximally from the piston head 326. The piston head 326 defines an annular channel 327 therein which surrounds the periphery of the piston head 326, wherein the channel 327 is configured to receive and maintain an O-ring "O" (FIG. 4) therein. The O-ring "O" fixes the bladder "B" to the piston head 326. The piston head 326, which is surrounded by the piston housing 330, and more particularly a piston housing wall 332, is coupled along a piston housing base 331. A gap is defined between the piston head 326 and the piston housing base 331 to permit expansion and contraction of the bladder "B" thereabout.

The shuttle 310 has a sensor mount 336 coupled to the head 316 of the shuttle 310. The sensor mount 336 has an anvil 337, and a chimney or stem 338 extending distally from the anvil 337. The chimney 338 has a threaded surface disposed distally along the exterior of the chimney 338, wherein the threaded surface is configured to mate with a threaded surface disposed about the opening of the head 316 of the shuttle 310. A bore 336a extends through the anvil 337 and the chimney 338 which is configured to receive a wired connector "SW" which couples to a sensor "S". The sensor "S" may be fixed to the anvil 337 of the sensor mount 336 or may freely float relative to the sensor mount 336. In embodiments, the sensor "S" may be integrated into the anvil 337.

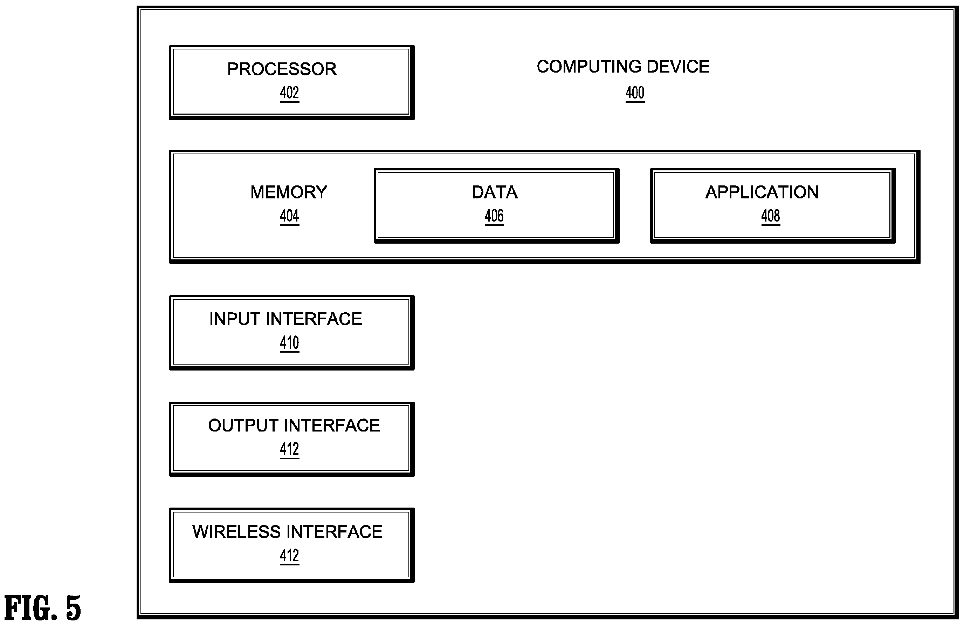

In the sensor "S" may include a light emitting diode or LED and/or a photo diode. The LED may be configured to transmit light toward the photo diode, the LED projecting light through target tissue toward the LED. Alternatively, or additionally, the LED may be configured to project light toward target tissue which, after striking the target tissue, is reflected and received by the photo diode. The light received by the photo diode may be received and transmitted to a computing device 400 (FIG. 5) to be analyzed.

Referring now to FIG. 4, illustrated is an alternate embodiment of the distal portion of the surgical device 10, including an alternate embodiment of the sensing assembly 300, referred to generally as surgical device 10' and sensing assembly 300'. While differences will be discussed herein between the surgical device 10 and the surgical device 10', a detailed description of previously described components will be omitted for purposes of clarity. It will be understood that, absent the differences discussed here, surgical device 10 and surgical device 10' are similar in many respects.

A compression housing 320' of the sensor assembly 300' includes a base 322' and a compression chamber wall 324. The compression chamber wall 324 extends distally from the base 322' of the compression housing 320' and encloses a cavity configured to receive a bladder "B" therein. The base 322' has an opening configured to receive a compression piston 325'. The compression piston 325' is coupled to a distal portion of the fluid conduit 302. A piston housing 330 of surgical device 10' is fixably joined to the base 322' of the compression housing 320' and is coupled to a proximal portion of the compression chamber wall 324 and the base 322'. The piston housing 330 may be coupled to the compression housing 320' via a friction fit, adhesives, or by any other suitable method of attachment.

The base 322' has a pair of openings 323 formed therein, in opposed relation to one another, which align with openings formed in an outer shaft 202' and transversely from a periphery of the base 322' inward. The openings 323 are configured to align with openings 329 of a shaft 328' of a piston 325'. The openings 323, 329 are configured to receive a bolt 340, or any other suitable threaded member, and rotatably engage threads disposed along the openings of the base 322' and the shaft 328' of the piston 325'.

Referring now to FIG. 5, illustrated is a schematic block diagram of a computing device 400 that may be employed according to various embodiments of the present disclosure. Though not explicitly shown in corresponding figures of the present application, the computing device 400, or one or more components thereof, may represent one or more components (e.g., a controller, input interface, output interface, and the like) of the surgical device 10. The components of the surgical device 10 may be disposed in and/or about the surgical device 10. Additionally, or alternatively, the components of the surgical device 10 may be located remote from the surgical device 10. The computing device 400 may include one or more processors 402, memories 404, input interfaces 410, output interfaces 412, wireless interfaces 414, or any desired subset of components thereof. The memory 404 includes non-transitory computer-readable storage media for storing data and/or software which include instructions that may be executed by the one or more processors 402. When executed, the instructions may cause the processor 402 to control operation of the computing device 400, e.g., reception and transmission of sensor signals transmitted and received during operation of the at least one sensor "S" located along the surgical device 10 (FIG. 3). In embodiments, the memory 404 may include one or more solid-state storage devices such as flash memory chips. Additionally, or alternatively, the memory 404 may include one or more mass storage devices in communication with the processor 402 through a mass storage controller and a communications bus (not shown). Although the description of computer readable media described in this disclosure refers to a solid-state storage device, it will be appreciated by one of ordinary skill that computer-readable media may include any available media that can be accessed by a processor 402. More particularly, computer readable storage media may include non-transitory, volatile, non-volatile, removable, non-removable media, and the like, implemented in any method of technology for storage of information such as computer readable instructions, data structures, program modules, or other suitable data access and management systems. Examples of computer-readable storage media include RAM, ROM, EPROM, EEPROM, flash memory, or other known solid state memory technology, CD-ROM, DVD, Blu-Ray, or other such optical storage, magnetic cassettes, magnetic tape, magnetic disk storage or other magnetic storage devices, or any other medium which may be used to store information and which can be accessed by computing device 400.

In embodiments, the memory 404 stores data 406 and/or one or more applications 408. Such applications 408 may include instructions which are executed on the one or more processors 402 of the computing device 400. The applications 408 may include instructions which cause an input interface 410 and/or an output interface 412 to receive and transmit sensor signals, respectively, to and from the surgical device 10. More particularly, as the at least one sensor "S" (*see* FIG. 3) senses one or more of the tissue properties discussed above, the at least one sensor "S" may, in response, transmit signals indicative of the measurements to the input interface 410, or by an external computing device 400. Once received by the input interface 410, the signals transmitted by the one or more sensors "S" may be stored in the at least one memory 404 of the computing device 400. Additionally, or alternatively, the computing device 400 may transmit the signals for analysis and/or display via the output interface 412. For example, the output interface 412 may transmit the sensor signals to a display device (not shown) either disposed on the surgical device 10 or located remotely relative to the surgical device 10. The memory 404 may further transmit and/or receive data via a wireless interface 414 via one or more wireless configurations, e.g., radio frequency, optical, Wi-Fi, Bluetooth (an open wireless protocol for exchanging data over short distances, using short length radio waves, from fixed and mobile devices, creating personal area networks (PANs), ZigBee® (a specification for a suite of high level communication protocols using small, low-power digital radios based on the IEEE 802.15.4-2003 standard for wireless personal area networks (WPANs)). Although depicted as a separate component, the wireless interface 414 may be integrated into the input interface 410 and/or the output interface 412.

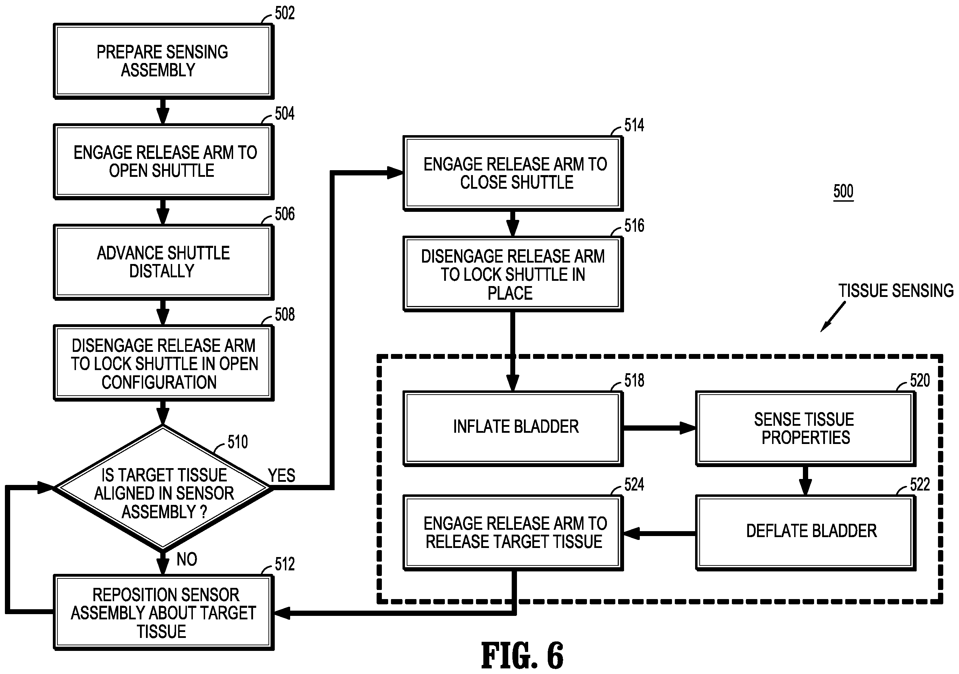

Referring now to FIG. 6, illustrated is a flow diagram detailing a method of operating the surgical device 10 (or surgical device 10') to sense one or more properties of target tissue. The method will be referred to generally as process 500.

In use, initially a clinician may prepare the surgical device 10 by installing a bladder "B" (FIG. 3A) on the piston head 326. To install the bladder "B" the clinician positions the bladder "B" about the piston head 326 and places an O-ring "O" (FIG. 4) about the channel surrounding the piston head 326. The clinician may additionally advance a fluid hose (not shown) through the access aperture 104 of the handle 100 and couple the fluid hose to the proximal portion of the fluid conduit 302 (block 502). In addition, a sensor wire "SW" may be advanced distally through the sensor mount 336 until the sensor "S" is pressed against the anvil 337 of the sensor mount 336.

Prior to insertion into a body cavity of a patient, the clinician may advance the shuttle 310 distal from the outer shaft 202 of the actuation assembly 200 to advance the sensing assembly 300 to the open configuration, if not already in the open configuration. To advance the shuttle 310, the clinician applies distal force to the knob 204 to advance the inner shaft 224 of the actuation assembly 200 until the engagement ring 228 engages the release 305. As the engagement ring 228 causes the engagement arm 306 of the release 305 to advance distally, the opening of the engagement arm 306 shifts relative to the fluid conduit 302 to release tension maintained by the engagement arm 306 (block 504). Once the tension applied by the engagement arm 306 is released, the expansion spring 318 may be compressed when in an expanded state (FIG. 3B) and the shuttle 310 advanced distally to urge the sensing assembly 300 from a closed configuration (FIG. 3B) to an opened configuration (FIG. 3A) (block 506). Once in the open configuration the clinician may discontinue the application of distal force to the knob 204, thereby allowing the engagement ring 228 to disengage the release 305 (block 508).

During a surgical procedure, the clinician may advance the distal portion of the surgical device 10 toward target tissue to be engaged by the sensing assembly 300. Once in close proximity to the target tissue, the clinician may position the sensing assembly 300 about the target tissue when determining where to measure one or more properties of the target tissue (block 510). More particularly, the clinician may position the target tissue in a cavity between the sensor "S" and the bladder "B". If, initially, the sensor assembly 300 is not positioned as desired about the target tissue (either when the sensor assembly 300 is in the open or closed configuration) the clinician may reposition, and if necessary re-engage the release 305, until the desired positioning of the sensing assembly 300 about the target tissue is achieved (block 512). After positioning the target tissue between the sensor "S" and the bladder "B" the clinician may again apply distal force to the knob 204 to cause the engagement ring 228 to mechanically engage the release 305 (block 514). As the engagement arm 306 is advanced distally by the engagement ring 228, tension applied by the engagement arm 306 to the fluid conduit 302 is released. The released tension enables the expansion spring 318 to cause the shuttle 310 to advance proximally, transitioning the sensing assembly 300 to a closed configuration (FIG. 3B). While in the closed configuration, a predetermined amount of pressure is exerted on the target tissue to maintain the position of the target tissue relative to the sensing assembly without occluding blood flow to the target tissue. In embodiments, it is contemplated that the pressure exerted by the expansion spring 318 may cause the blood flow of the target tissue to be partially or completely occluded. The knob 204 may be released and/or advanced proximally, thereby allowing the engagement arm 306 to disable motion of the shuttle 310 (block 516).

Once the sensing assembly 300 is in a closed configuration about the target tissue, the bladder "B" may receive fluid (e.g., air) and be caused to expand (block 518). If the pressure sensor (not shown) detects that pressure has not increased beyond a predetermined threshold (e.g., the engagement arm 306 was disengaged, allowing the shuttle 310 to move relative to the surgical device 10) the computing device 400 may cause an error warning to appear on a display device or otherwise output feedback (e.g., an audio signal) indicating sufficient pressure cannot be established in the bladder "B" to allow the sensor to operate. As the bladder "B" expands, the bladder "B" presses the target tissue against the sensor "S", compressing the target tissue to occlude blood flow. The sensor "S" may be selected from one or more sensors including piezoresistive force sensors, optical sensors, temperature sensors, pressure sensors, blood oxygen saturation sensors (SpO2), tissue oxygen saturation sensors (StO2), and/or impedance sensors. As noted above, the sensor "S" may also include an LED and/or a photo diode, the LED configured to transmit light toward target tissue and the photo diode configured to receive the light transmitted by the LED. As sensor measurements are taken by the sensor "S" the sensor measurements may be transmitted to a computing device 400 or controller (FIG. 5) for analysis (block 520).

After sensing is completed, the clinician may cause the bladder "B" to deflate prior to removal of the surgical device 10 from the body of the patient (block 522). Additionally, or alternatively, the clinician may apply distal force to the knob 204 to cause the engagement ring 228 to engage the release 305. More particularly, the clinician may apply distal force to release pressure against the fluid conduit 302 exerted by the engagement arm 306. Additional force may be applied to the knob 204 by the clinician to compress the expansion spring 318 to urge the shuttle 310 distally to release the target tissue (block 524). Once the target tissue is released, the clinician may remove the surgical device 10. The clinician may repeat this procedure to obtain multiple sensor measurements about multiple portions of target tissue.

For a detailed description of systems and methods of sensing tissue properties during laparoscopic surgical procedures, reference may be made to U.S. Provisional Patent Application No.: 62/661821 filed April 24, 2018, the entire contents of which are hereby incorporated by reference in their entirety.

Although illustrative embodiments of the present disclosure have been described herein, it is understood that the disclosure is not limited to those precise embodiments, and that various other changes and modifications may be affected therein by one skilled in the art without departing from the scope or spirit of the present disclosure. All such changes and modifications are intended to be included within the scope of the present disclosure.

The invention may be described by reference to the following numbered paragraphs:-
1. A surgical device for sensing a tissue property, the surgical device comprising:
   a handle;
   an outer shaft coupled to the handle;
   an inner shaft slidably coupled to the outer shaft; and
   a sensing assembly coupled to a distal portion of the outer shaft, the sensing assembly including:
      a shuttle having an anvil and a sensor disposed on the anvil;
      a compression housing coupled to the distal portion of the outer shaft, the compression housing located proximal to the anvil;
      an expansion spring disposed between the shuttle and the compression housing; and
      a release coupled to the shuttle and biased to engage a fluid conduit, the release configured to fix the position of the shuttle relative to the outer shaft.
2. The surgical device of paragraph 1, further comprising a bladder coupled to the fluid conduit, the bladder configured to apply pressure to target tissue when fluid is introduced to the bladder.
3. The surgical device of paragraph 2, wherein the shuttle is configured to transition from an open configuration to a closed configuration in response to longitudinal movement of the inner shaft.
4. The surgical device of paragraph 1, wherein the release is configured to apply pressure to the fluid conduit to lock the shuttle in position relative to the outer shaft.
5. The surgical device of paragraph 4, wherein the pressure applied by the release to the fluid conduit is greater than the pressure exerted by the expansion spring to the shuttle.
6. The surgical device of paragraph 1, wherein engagement of the release by the inner shaft when the shuttle is advanced distally causes the shuttle to advance proximally.
7. The surgical device of paragraph 6, further comprising a bladder coupled to the fluid conduit, the bladder configured to expand and compress target tissue when the shuttle is in a closed configuration.
8. A method of sensing a tissue property with a tissue property sensing device, the method comprising:
   positioning a sensing assembly of a tissue property sensing device about target tissue;
   engaging a release arm to transition the sensing assembly from an open configuration to a closed configuration;
   compressing the target tissue disposed in the sensing assembly; and
   sensing a tissue property.
9. The method of paragraph 8, wherein engaging the release arm causes an expansion spring to apply force to a shuttle to move the shuttle from a distal position relative to the tissue property sensing device to a proximal position.
10. The method of paragraph 8, further comprising disengaging the release arm after the sensing assembly is transitioned to the closed configuration.
11. The method of paragraph 10, wherein disengaging the release arm causes the sensing assembly to lock in the closed configuration.
12. The method of paragraph 11, further comprising:
   engaging the release arm after sensing the tissue property; and
   transitioning the sensing assembly from the closed configuration to the open configuration,
   wherein engaging the release arm causes the sensing assembly to unlock.
13. The method of paragraph 12, further comprising decompressing the target tissue prior to transitioning the sensing assembly from the closed configuration to the open configuration.
14. The method of paragraph 8, wherein compressing includes increasing pressure in a balloon fixed to the sensing assembly.
15. The method of paragraph 8, further comprising:
   locking the sensing assembly when in the closed configuration,
   wherein compressing includes delivering fluid to the balloon to increase the pressure of the balloon when the sensing assembly is locked in the closed configuration.

## Claims

1. A surgical device for sensing a tissue property, the surgical device comprising:
a handle;
an outer shaft coupled to the handle;
an inner shaft slidably coupled to the outer shaft; and
a sensing assembly coupled to a distal portion of the outer shaft, the sensing assembly including:
a shuttle having an anvil and a sensor disposed on the anvil;
a compression housing coupled to the distal portion of the outer shaft, the compression housing located proximal to the anvil;
an expansion spring disposed between the shuttle and the compression housing; and
a release coupled to the shuttle and biased to engage a fluid conduit, the release configured to fix the position of the shuttle relative to the outer shaft.

2. The surgical device of claim 1, further comprising a bladder coupled to the fluid conduit, the bladder configured to apply pressure to target tissue when fluid is introduced to the bladder.

3. The surgical device of claim 2, wherein the shuttle is configured to transition from an open configuration to a closed configuration in response to longitudinal movement of the inner shaft.

4. The surgical device of any preceding claim, wherein the release is configured to apply pressure to the fluid conduit to lock the shuttle in position relative to the outer shaft.

5. The surgical device of claim 4, wherein the pressure applied by the release to the fluid conduit is greater than the pressure exerted by the expansion spring to the shuttle.

6. The surgical device of any preceding claim, wherein engagement of the release by the inner shaft when the shuttle is advanced distally causes the shuttle to advance proximally.

7. The surgical device of claim 6, further comprising a bladder coupled to the fluid conduit, the bladder configured to expand and compress target tissue when the shuttle is in a closed configuration.
